# EUROPÄISCHE PATENTSCHRIFT

(11) **EP 0 574 783 B1**
(45) Veröffentlichungstag und Bekanntmachung des Hinweises auf die Patenterteilung: **06.10.1999**
(21) Anmeldenummer: 93109092.2
(22) Anmeldetag: 07.06.1993
(51) Int. Cl.: C07D 471/04

(54) **Asymmetrische Hydrierung**
Asymmetric hydrogenation
Hydrogénation asymétrique

(30) Priorität: 19.06.1992 CH 194492; 18.03.1993 CH 82693
(43) Veröffentlichungstag der Anmeldung: 22.12.1993
(73) Patentinhaber: F. HOFFMANN-LA ROCHE AG, 4002 Basel (CH)
(72) Erfinder: Broger, Emil Albin, CH-4312 Magden (CH)
(74) Vertreter: Cottong, Norbert A.

(56) Entgegenhaltungen:
- EP-A- 0 158 875
- EP-A- 0 540 956
- TETRAHEDRON Bd. 47, Nr. 26 , 24. Juni 1991 , OXFORD GB Seiten 4645 - 4664 RINO A. BIT ET AL 'A Dieckmann/ring expansion approach to tetrahydropyrido- and tetrahydroazepino-[1,2-a]indoles'

## Beschreibung

Die vorliegende Erfindung betrifft ein neues katalytisches Verfahren zur Herstellung von Verbindungen der allgemeinen Formel 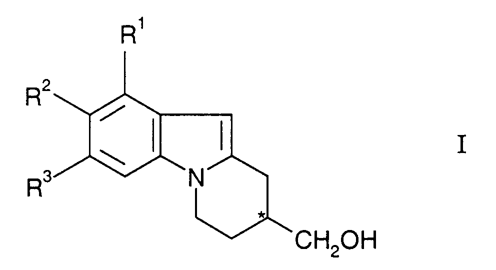
worin R¹, R² und R³ unabhängig voneinander Wasserstoff, Halogen, C₁-C₇ Alkyl, C₁-C₇ Halogenalkyl, Hydroxy, C₁-C₇ Alkoxy, C₁-C₇ Alkylthio, C₁-C₇ Alkylsulfinyl, C₁-C₇ Alkylsulfonyl, Nitro, Amino oder Acylamino bedeuten und * das chirale Zentrum bezeichnet,
in der (S)- oder (R)-Form, die wertvolle Zwischenprodukte sind, z.B. für die Herstellung von pharmazeutischen Wirkstoffen.

Das erfindungsgemässe Verfahren ist dadurch gekennzeichnet, dass man das entsprechende 6,7-Dihydropyridol [1,2-a]indol-8-methanol der allgemeinen Formel 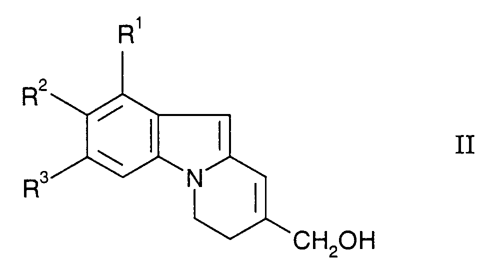
worin R¹, R² und R³ die oben angegebenen Bedeutungen besitzen,
in Gegenwart eines optisch aktiven Rhodium-Diphosphin-Komplexes asymmetrisch hydriert, wobei man als optisch aktiven Rhodium-Diphosphin-Komplex einen Komplex der allgemeinen Formel

[Rh(X¹)(Y¹)(L¹)m]n III'

verwendet, worin
- X¹: Halogenid, ein Anion R⁴-COO-, ein Anion R⁴-SO₃-, 1,3-Diketonat oder gegebenenfalls mit C₁-C₄ Alkyl und/oder Halogen einfach oder mehrfach substituiertes Phenolat,
- Y¹: einen optisch aktiven atropisomeren Diphosphinliganden der allgemeinen Formel 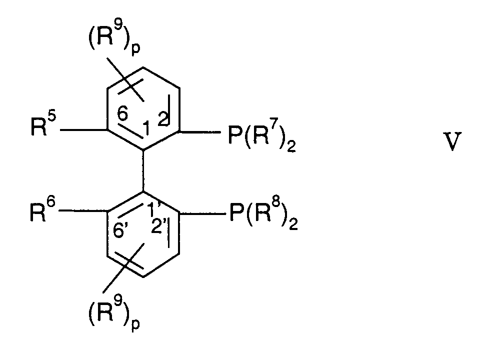 oder der allgemeinen Formel 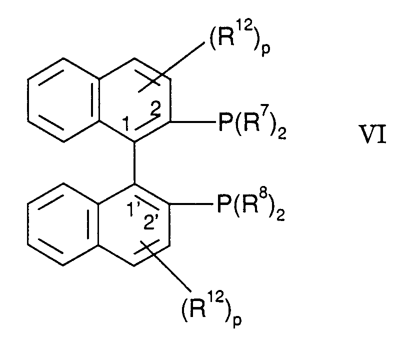
- L¹,: oder jedes L¹ unabhängig, einen olefinischen Liganden, ein Nitril oder ein Lösungsmittelmolekül,
- m: 0,1 oder 2,
- n: 1 oder 2,
- R⁴: C₁-C₄ Alkyl, halogeniertes C₁-C₄ Alkyl oder Aryl,
- R⁵ und R⁶: unabhängig voneinander C₁-C₄ Alkyl, C₁-C₄ Alkoxy, Di(C₁-C₄ Alkyl)amino, Hydroxy, geschütztes Hydroxy, Hydroxymethyl oder geschütztes Hydroxymethyl,
oder
- R⁵ und R⁶: zusammen eine zweiwertige Gruppe 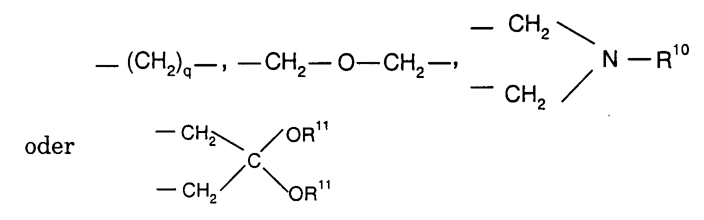
- R⁷ und R⁸: unabhängig voneinander C₁-C₄ Alkyl, C₃-C₇-Cycloalkyl, gegebenenfalls substituiertes Phenyl, einen fünfgliedrigen Heteroaromaten oder eine Gruppe der Formel 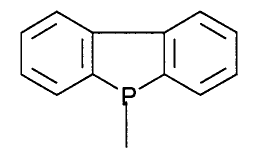
- R⁹: C₁-C₄ Alkyl oder C₁-C₄ Alkoxy,
- R¹⁰: C₁-C₄ Alkyl, gegebenenfalls substituiertes Phenyl oder gegebenenfalls substituiertes Benzyl,
- R¹¹: C₁-C₄ Alkyl oder beide R¹¹ zusammen Di- oder Trimethylen,
- R¹²: Halogen, Hydroxy, Methyl, Aethyl, Amino, Acetamido, Nitro oder Sulfo,
- p: Null oder die Zahl 1, 2 oder 3
- und q: die Zahl 3, 4 oder 5 bedeuten,

wobei der Begriff 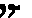 Acyl" in 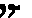 Acylamino" eine Gruppe bezeichnet, die von einer Alkankarbonsäure mit bis zu 7 Kohlenstoffatomen abgeleitet ist,
der Begriff 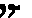 1,3-Diketonat" eine Verbindung der Formel R¹⁵-CO-CH₂-CO-R¹⁵ bezeichnet, in denen R¹⁵ C₁-C₄ Alkyl oder Aryl gemäss nachstehender Definition ist,
der Begriff 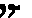 Aryl" Phenyl, Biphenyl oder Napthyl bedeutet, das unsubstituiert oder einfach oder mehrfach mit gleichen oder verschiedenen C₁-C₄ Alkylgruppen und/oder Halogenatomen substituiert ist,
der Begriff 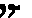 geschütztes Hydroxy" oder 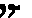 geschütztes Hydroxymethyl" mit ätherbildenden Gruppen geschützte Hydroxygruppen bedeutet,
der Begriff 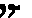 ggf. Substituiertes Phenyl" bzw. 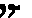 ggf. Substituiertes Benzyl" bezüglich des Substituenten des Phenylteils Fluor, C₁-C₄ Alkyl, C₁-C₄ Alkoxy, Di(C₁-C₄ Alkyl)amino, Di(C₁-C₄ Alkyl)silyl und Phenyl bedeutet,
der Begriff 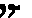 fünfgliedriger Heteroaromat" für einen Substituenten der Formel 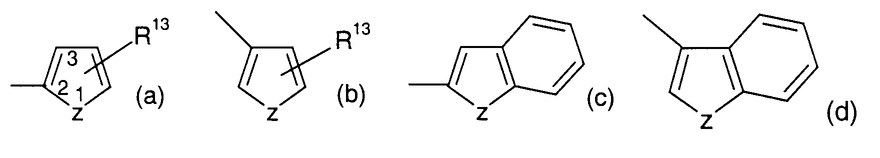
steht, worin Z Sauerstoff, Schwefel oder NR¹⁴; R¹³ C₁-C₄ Alkyl oder C₁-C₄ Alkoxy und R¹⁴ C₁-C₄ Alkyl bedeuten.

EP-A-0 158 875 offenbart chirale Rhodium-diphosphinkomplexe als Katalysatoren bei asymmetrischen Hydrierungen u.a. von α,β-ungesättigten Säuren und Estern, von α-Ketocarbonsäuren und Estern und von α-Keto-Lactonen (siehe S.2, Z.10-12, und S. 8, 1. Absatz). Spezifisch ist u.a. die Hydrierung von Dihydro-4,4-dimethyl-2,3-furandion beschrieben (siehe z.B. Beispiel 2).

Bit et al. (Tetrahedron, 47(26), (1991), S. 4645-4664) offenbaren die Herstellung von u.a. Tetrahydropyrido[1,2-a]indolen durch Dieckmann/Ringexpansion-Reaktionen. Dabei wird u.a. die Herstellung von 6,7,8,9-Tetrahydropyrido[1,2-a]indol-8-methanol der Formel 7a durch Hydrierung des entsprechenden Esters der Formel 6a (siehe S. 4646 und 4654) offenbart.

EP-A-0 540 956 offenbart die Herstellung von Maleimidderivaten der Formel I. Dabei wird u.a. die Herstellung von (S)-8-(Hydroxymethyl)-6,7,8,9-tetrahydropyrido[1,2-a]indolen durch die Hydrierung des entsprechenden Esters beschrieben (siehe Beispiel 2). Das enthaltene Produkt wird zur Herstellung von (S)-8-(Acetoxymethyl)-6,7,8,9-tetrahydropyrido[1,2-a]indol verwendet.

Die Ausgangsmaterialien der Formel II sind neu und bilden einen weiteren Gegenstand der vorliegenden Erfindung.

Im Rahmen der obigen Definition der Formeln I und II sind unter dem Ausdruck 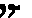 Alkyl" Alkylgruppen zu verstehen, die je nach Anzahl der Kohlenstoffatome geradkettig oder verzweigt sein können. Beispiele solcher Alkylgruppen sind Methyl, Aethyl, Propyl, Isopropyl, n-Butyl, Isobutyl, tert. Butyl, n-Pentyl, tert. Pentyl, Neopentyl, n-Hexyl, n-Heptyl und dergleichen. Der Ausdruck 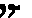 Halogenalkyl", 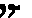 Alkoxy", 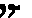 Alkylthio", 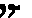 Alkylsulfinyl" bzw. 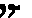 Alkylsulfonyl" bedeutet derartige Gruppen, in denen der Alkylteil die vorhergehende Bedeutung hat. Der Ausdruck 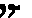 Halogen" bedeutet Fluor, Chlor, Brom oder Jod. Die Halogenalkylgruppe kann einen oder mehrere Halogensubstituenten aufweisen. Beispiele solcher Gruppen sind Chlormethyl und Trifluormethyl. Der Ausdruck 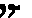 Acyl" der Gruppe 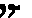 Acylamino" bezeichnet eine Gruppe, die von einer Alkancarbonsäure mit bis zu 7, vorzugsweise bis zu 4, Kohlenstoffatomen, wie beispielsweise Formyl, Acetyl, Propionyl oder Butyryl, oder von einer aromatischen Carbonsäure, z.B. Benzoyl, abgeleitet ist.

In der obigen Definition der Formel III' ist unter dem Ausdruck 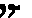 olefinischer Ligand", z.B. Aethylen, Propylen, Cycloocten, 1,5-Hexadien, Norbornadien, 1,5-Cyclooctadien und dergleichen zu verstehen, ein Nitril z.B. Acetonitril, Benzonitril und dergleichen, und ein Lösungsmittelmolekül z.B. dasjenige des Lösungsmittels, in dem der Rhodium-Katalysator hergestellt wird. L¹ kann bei der Hydrierung ausgetauscht werden. Falls mehr als ein L¹ vorhanden ist (m = 2), können diese auch voneinander verschieden sein.

Im Rahmen der obigen Definition der Formeln III', V und VI und in den nachfolgenden Erläuterungen bedeutet der Ausdruck 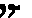 Halogenid" bzw. 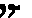 Halogen" Fluor, Chlor, Brom oder Jod. Der Ausdruck 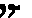 C₁-C₄ Alkyl" bedeutet geradkettige oder verzweigte Alkylgruppen mit 1 bis 4 Kohlenstoffatomen, wie Methyl, Aethyl, Propyl, Isopropyl, n-Butyl, Isobutyl und tert. Butyl. Der Ausdruck 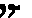 C₁-C₄ Alkoxy" bedeutet Gruppen, in denen der Alkylrest die vorhergehende Bedeutung hat. Dies gilt auch für andere 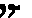 C₁-C₄ Alkyl" enthaltende Gruppen, wie halogeniertes C₁-C₄ Alkyl und Di(C₁-C₄ Alkyl)amino. Unter der soeben erwähnten Gruppe 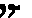 halogeniertes C₁-C₄ Alkyl" sind C₁-C₄ Alkylgruppen zu verstehen, die einfach oder mehrfach mit gleichen oder verschiedenen Halogenatomen substituiert sind, und zwar insbesondere mit Fluor und/oder Chlor. Vorzugsweise befindet sich ein Halogenatom in α-Stellung (am Rest R⁴) zur ―COO⁻. Bevorzugte halogenierte C₁-C₄ Alkylgruppen sind perchlorierte und perfluorierte C₁-C₄ Alkylgruppen, z.B. Trichlormethyl bzw. Pentafluoräthyl. Der Ausdruck 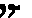 Aryl" bedeutet insbesondere Phenyl, Biphenyl oder Naphthyl, das unsubstituiert oder einfach oder mehrfach mit gleichen oder verschiedenen C₁-C₄ Alkylgruppen und/oder Halogenatomen substituiert ist, wobei bevorzugte halogenierte Arylgruppen R⁴ Perchlorphenyl und Perfluorphenyl sind. Falls der Rest R⁷, R⁸ oder R¹⁰ gegebenenfalls substituiertes Phenyl oder, bezüglich R¹⁰ allein, gegebenenfalls substituiertes Benzyl bedeutet, kommen hier als Substituenten (im Falle von Benzyl, für dessen Phenylteil) insbesondere Fluor, C₁-C₄ Alkyl- oder Alkoxygruppen (vorzugsweise Methyl bzw. Methoxy), Di(C₁-C₄ Alkyl)amino (vorzugsweise Dimethylamino), Tri(C₁-C₄ Alkyl)silyl (vorzugsweise Trimethylsilyl) und Phenyl in Frage. Als Schutzgruppen für die Hydroxy- bzw. Hydroxymethylgruppe (R⁵ und/oder R⁶ als geschütztes Hydroxy oder geschütztes Hydroxymethyl) kommen insbesondere die üblichen Aetherbildenden Gruppen, wie z.B. Benzyl, Allyl, Benzyloxymethyl, niederes Alkoxymethyl oder auch 2-Methoxyäthoxymethyl und dergleichen in Betracht. Der Ausdruck 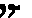 fünfgliedriger Heteroaromat" (R⁷, R⁸) steht für einen Substituenten der Formel 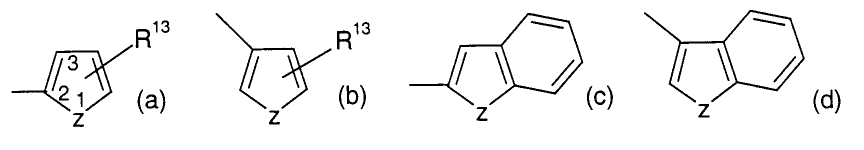
worin Z Sauerstoff, Schwefel oder NR¹⁴; R¹³ Wasserstoff, C₁-C₄ Alkyl (insbesondere Methyl) oder C₁-C₄ Alkoxy (insbesondere Methoxy); und R¹⁴ C₁-C₄ Alkyl (insbesondere Methyl) bedeuten.

Falls p in der Formel VI eine Zahl 1 bis 3 bedeutet, befinden sich die beiden bzw. zwei der Reste R¹² vorzugsweise in 5,5'-Stellung.

Unter dem Ausdruck 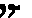 1,3-Diketonat" (X¹ der Formel III') sind insbesondere Reste zu verstehen, die von aliphatischen und aromatischen Diketonen der Formel R¹⁵-CO-CH₂-CO-R¹⁵ abgeleitet sind, in denen R¹⁵ C₁-C₄ Alkyl oder Aryl bedeutet.

In der Formel III' bedeutet X¹ vorzugsweise Halogenid, insbesondere das Bromid-Ion.

Bevorzugte Liganden Y¹ sind diejenigen der Formel V, worin R⁵ und R⁶ gleich sind und jeweils C₁-C₄ Alkyl oder C₁-C₄ Alkoxy bedeuten, oder alternativ zusammen die Gruppe -CH₂-O-CH₂- bedeuten, und R⁷ und R⁸ gleich sind und jeweils unsubstituiertes oder ein- oder mehrfach in der 3-, 4-und/oder 5-Stellung substituiertes Phenyl bedeuten und R⁹ Methyl oder Methoxy und p 0 oder 1 bedeuten. Falls in dieser Formel p die Zahl 1 bedeutet, befindet sich jeder Substituent R⁹ vorzugsweise in para-Stellung zum jeweiligen Phosphoratom.

Die erfindungsgemässe asymmetrische Hydrierung der Verbindung der Formel II zur Verbindung der Formel I kann in einem geeigneten, unter den Reaktionsbedingungen inerten organischen Lösungsmittel erfolgen. Als derartige Lösung kommen insbesondere aliphatische Ester, wie beispielsweise Aethylacetat; cyclische Aether, wie beispielsweise Tetrahydrofuran und Dioxan; und aromatische Kohlenwasserstoffe, wie beispielsweise Benzol und Toluol; und auch Gemische obiger Lösungsmittel in Betracht. Die Hydrierung wird zweckmässigerweise bei Temperaturen zwischen etwa 20°C und etwa 140°C, vorzugsweise im Temperaturbereich von etwa 60°C bis etwa 120°C, und bei einem Druck von etwa 1 bis etwa 100 bar, vorzugsweise von etwa 2 bis etwa 60 bar, durchgeführt. Das Molverhältnis von Rhodium zum Diphosphinliganden Y¹ im Komplex der Formel III' liegt zweckmässigerweise zwischen etwa 0.05:1 und etwa 5:1, vorzugsweise zwischen etwa 0,5:1 und etwa 2:1. Was das Molverhältnis von Rhodium zum koordinierenden anionischen Liganden X¹ im Komplex der Formel III' anbelangt, so liegt dieses zweckmässigerweise zwischen etwa 0,01:1 und etwa 20:1, vorzugsweise zwischen etwa 0,5:1 und etwa 10:1. Das prozentuale Molverhältnis von Rhodium im Rhodium-Katalysator zu der zu hydrierenden Verbindung der Formel II (dem 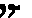 Substrat") liegt zweckmässigerweise zwischen etwa 0,001 und etwa 5 [entspricht einem Molverhältnis Substrat:Katalysator (S/C) von etwa 100.000 bis etwa 20], vorzugsweise zwischen etwa 0,002 und etwa 0,2 (S/C etwa 50.000 bis 500).

Falls eine Verbindung der Formel I herzustellen ist, die sich in der (S)-Form befindet, so setzt man als Katalysator einen optisch aktiven Rhodium-Diphosphin-Komplex der Formel III' ein, worin der optisch aktive atropisomere Diphosphinligand Y¹ in der (R)-Form vorliegt. Zur Herstellung der (R)-Verbindungen der Formel I wird der entsprechende Diphosphinligand Y¹ in der (S)-Form verwendet.

Das erfindungsgemässe Verfahren erlaubt die Herstellung der Verbindungen der Formel I in hoher optischer Reinheit. Es wird besonders bevorzugt für die Herstellung von (S)-6,7,8,9-Tetrahydropyrido[1,2-a]indol-8-methanol [(S)-Verbindung der Formel I, in der R¹, R² und R³ jeweils Wasserstoff bedeutet] eingesetzt. Diese Verbindung ist ein wertvolles Zwischenprodukt in der Synthese von (S)-3-{6,7,8,9-Tetrahydro-8-[(dimethylamino)methyl]pyrido[1,2-a]indol-10-yl}-4-(1-methyl-3-indolyl)-1H-pyrrol-2,5-dion-Hydrochlorid, eines wertvollen pharmazeutischen Wirkstoffs für die Behandlung oder Prophylaxe von Krankheiten, speziell von inflammatorischen, immunologischen, onkologischen, bronchopulmonären und kardiovasculären Krankheiten.

Die optisch aktiven Rhodium-Diphosphin-Komplexe der Formel III', sind entweder bekannt oder können in an sich bekannter Weise hergestellt werden, z. B. indem man einen optisch aktiven atropisomeren Diphosphinliganden der Formel V oder VI mit einer Verbindung, welche Rhodium abgeben kann, in einem geeigneten, inerten organischen oder wässrigen Lösungsmittel umsetzt. Als geeignete Rhodium abgebende Verbindungen können beispielsweise genannt werden organische Rhodium-Komplexe mit Aethylen, Propylen und dergleichen, sowie mit Dienen, z. B. (Z,Z)-1,5-Cyclooctadien, 1,5-Hexadien und Bicyclo[2.2.1]hepta-2,5-dien, welche mit Rhodium leicht lösliche Komplexe bilden. Bevorzugte Rhodium abgebende Verbindungen sind z. B. Di-µ-chloro-bis[η⁴-(Z,Z)-1,5-cyclooctadien]dirhodium(I), Di-µ-chloro-bis[η⁴-norbornadien]dirhodium(I), Di-µ-trifluoracetato-bis[η⁴-(Z,Z)-1,5-cyclooctadien]dirhodium(I), Bis[η⁴-(Z,Z)-1,5-cyclooctadien]rhodium-tetrafluorborat und Bis[η⁴-(Z,Z)-cyclooctadien]rhodium-perchlorat.

Die Liganden der Formeln V und VI selbst sind bekannt, z. B. aus den europäischen Patentpublikationen 104.375 und 398.132 bzw. aus der japanischen Patentpublikation (Kokai) 136. 605/1978, oder können in Analogie zu der Herstellung der bekannten Liganden erhalten werden. Diese Publikationen beinhalten Methoden zur Herstellung derjenigen Liganden der Formeln V und VI, in denen R⁷ und R⁸ gleich sind. Diejenigen Verbindungen, worin R⁷ und R⁸ voneinander verschieden sind, können analog hierzu erhalten werden, allerdings in zwei Stufen, beispielsweise gemäss folgendem Reaktionsschema: 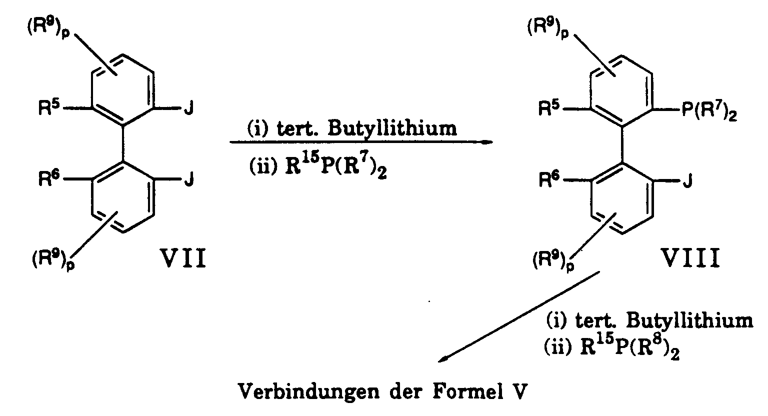 , worin R⁵, R⁶, R⁷, R⁸, R⁹ und p die oben angegebenen Bedeutungen besitzen und R¹⁵ eine Abgangsgruppe bedeutet, z. B. Halogen, insbesondere Chlor oder Brom; oder Alkoxy, insbesonder Methoxy oder Aethoxy. Um zu gewährleisten, dass jeweils nur ein Jodatom durch ein Lithiumatom unter Verwendung des Alkyllithiums (tert. Butyllithium) ersetzt wird, werden die entsprechenden Reaktionen zweckmässigerweise mit etwa äquivalenten Mengen an Reaktionspartnern durchgeführt.

Bei der Durchführung der erfindungsgemässen asymmetrischen Hydrierung kann der optisch aktive Rhodium-Diphosphin-Komplex der Formel III' zuerst hergestellt werden, und dann eine Lösung der zu hydrierenden Verbindung der Formel II zugegeben werden. Alternativ kann der Rhodium-Katalysator jedoch in situ hergestellt werden, und zwar wunschgemäss in Gegenwart oder in Abwesenheit der zu hydrierenden Verbindung.

Die in dem erfindungsgemässen Verfahren als Ausgangsmaterialien verwendeten 6,7-Dihydropyridol[1,2-a]indol-8-methanole der allgemeinen Formel II sind neu und bilden einen weiteren Gegenstand der vorliegenden Erfindung. Diese Verbindungen können beispielsweise gemäss folgendem Reaktionsschema hergestellt werden:

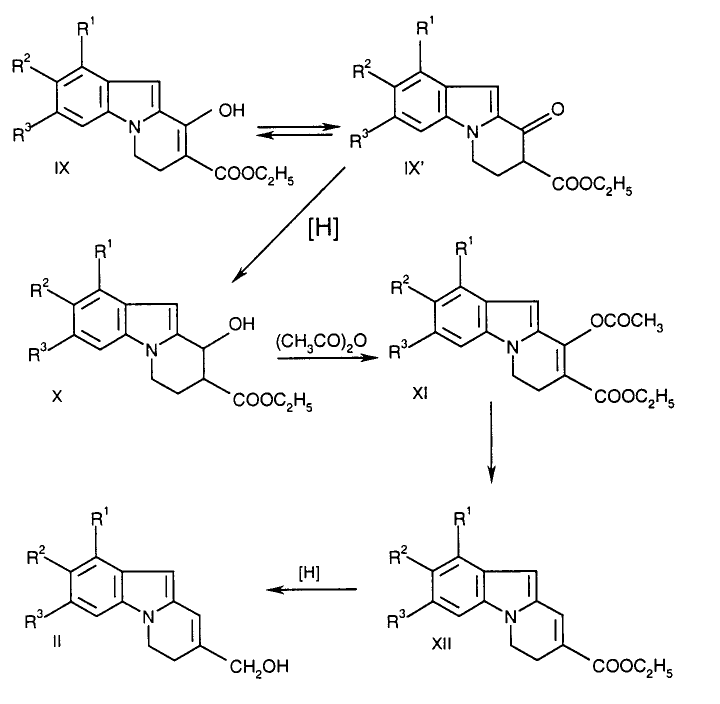

Die Umwandlung des gegebenenfalls substituierten 6,7-Dihydro-9-hydroxypyrido[1,2-a]indo-8-carbonsäure-äthylesters der Formel IX oder des entsprechenden β-Keto-esters der Formel IX' in die entsprechende 6,7,8,9-Tetrahydroverbindung der Formel X erfolgt zweckmässigerweise unter Verwendung von Ammoniumformiat als Reduktionsmittel in Gegenwart von Palladium auf Kohle als Katalysator. Die Reduktion wird zudem zweckmässigerweise in einem organischen Lösungsmittel, insbesondere Aethanol, und bei der Rückflusstemperatur des diesbezüglichen Reaktionsgemisches durchgeführt. Um Sauerstoff soweit wie möglich vom reduktiven Reaktionssystem auszuschliessen, erfolgt die Reaktion zweckmässigerweise unter einer Inertgasatmosphäre, vorzugsweise Stickstoff.

Bei der nächsten Reaktionsstufe handelt es sich um eine Acetylierung, die zweckmässigerweise mittels Essigsäureanhydrid oder Acetylchlorid vorgenommen wird. Man verwendet zweckmässigerweise ein organisches Lösungsmittel, wie beispielsweise einen aliphatischen Ester, z. B. Aethylacetat, sowie eine für Acetylierungen übliche Base, oder noch geeigneter, ein organisches Lösungsmittel, das gleichzeitig als Base dient, wie beispielsweise Pyridin oder ein substituiertes Derivat davon, z. B. ein Lutidin oder ein Collidin. Die Acetylierung des gegebenenfalls substituierten 6,7,8,9-Tetrahydro-9-hydroxypyrido[1,2-a]indol-8-carbonsäure-äthylesters X erfolgt zweckmässigerweise bei Temperaturen zwischen ca. 40°C und ca. 90°C.

Der resultierende 9-Acetoxy-6,7,8,9-tetrahydropyrido[1,2-a]indo-8-carbonsäure-äthylester XI wird dann unter Verwendung einer starken Base, z. B. 1,1,3,3-Tetramethylguanidin oder Diazobicycloundecan, einer Eliminierungsreaktion zum entsprechenden 6,7-Dihydropyrido[1,2-a]indol-8-carbonsäure-äthylester der Formel XII unterworfen. Diese Eliminierung erfolgt zweckmässigerweise in einem organischen Lösungsmittel, wie beispielsweise einem aromatischen Kohlenwasserstoff, z. B. Benzol oder Toluol, oder einem aliphatischen Alkohol, z. B. Aethanol, und je nach verwendeter Base bei Temperaturen zwischen ca. 40°C und ca. 90°C.

Die Reduktion des in der bisherigen Reaktionsstufe hergestellten 6,7-Dihydropyrido[1,2-a]indo-8-carbonsäure-äthylesters der Formel XII zur entsprechenden 8-Methanol-Verbindung, d. h. zum für das erfindungsgemässe Verfahren benötigten Ausgangsmaterial, der Formel II erfolgt unter Verwendung eines (gemischten) Reduktionsmittels, das Lithiumionen und Borhydridionen enthält, wie beispielsweise Lithiumchlorid und Natriumborhydrid, Lithiumbromid und Kaliumborhydrid. Alternativ kann man zu diesem Zweck ein Reduktionsmittel des Typs Lithiumborhydrid oder des Typs Diisobutylaluminiumhydrid verwenden. Die Umsetzung wird zudem in einem geeigneten Lösungsmittel durchgeführt, wie einem aliphatischen oder cyclischen Aether, z. B. Diglym bzw. Tetrahydrofuran oder Dioxan. Man arbeitet im Falle der meisten in Frage kommenden Reduktionsmittel bei Temperaturen um Raumtemperatur, bei Verwendung von Lithiumborhydriden jedoch eher bei der Rückflusstemperatur des Reaktionsgemisches.

Die folgenden Beispiele dienen zur Erläuterung der Erfindung. In diesen Beispielen haben die verwendeten Abkürzungen folgende Bedeutung:
- DC: Dünnschichtchromatographie
- GC: Kapillar-Gaschromatographie
- e.e.: Enantiomeric excess (Enantiomeren-Überschuss). Der e.e. der Hydrierprodukte wird durch GC-Analyse der aus Camphansäurechlorid (Fluka) hergestellten diastereomeren Ester bestimmt.
- BIPHEMP: (6,6'-Dimethylbiphenyl-2,2'-diyl)bis(diphenylphosphin)
- MeOBIPHEP: (6,6'-Dimethoxybiphenyl-2,2'-diyl)bis(diphenylphosphin)

### Beispiel 1

### Herstellung von (R)-6,7,8,9-Tetrahydropyrido[1,2-a]indol-8-methanol

a) In einer Glove-Box (Sauerstoffgehalt <1 ppm) werden in einem 50 ml-Glaskolben 12,25 mg (0,025 mMol) Di-µ-chlor-bis-(1,5-cyclooctadien)di rhodium(I) und 29,25 mg (0,050 mMol)(S)-MeOBIPHEP in 50 ml Toluol suspendiert. Die Suspension wird anschliessend 10 Minuten gerührt, wobei sich eine klare, gelbe Lösung (die Katalysatorlösung) bildet.
b) In einer Glove-Box (Sauerstoffgehalt <1 ppm) wird der Glaseinsatz eines 30 ml-Autoklaven mit 0,20 g (1,0 mMol) 6,7-Dihydropyrido[1,2-a]indol-8-methanol, 8 ml Toluol und 2 ml der oben erwähnten Katalysatorlösung beladen. Die Hydrierung wird bei 80°C, einem Anfangsdruck von 60 bar H₂ und unter intensivem Rühren durchgeführt. Nach 18 Stunden Hydrierzeit beträgt der Umsatz gemäss GC 99,9%. Die hellgelbe Hydrierlösung wird eingedampft, der Rückstand in Diäthyläther gelöst, und die Lösung zur Abtrennung des Katalysators durch eine Säule mit 5 g Kieselgel fliessen gelassen. Nachwaschen der Säule mit Diäthyläther, Eindampfen des Eluates und Trocknen des hellbraunen, kristallinen Rückstandes liefern 0,20 g (100%) (R)-6,7,8,9-Teträhydropyrido[1,2-a]indol-8-methanol; GC 100 Flächen-%; 93,7 % e.e.

### Beispiel 2

### Herstellung von (S)-6,7,8,9-Tetrahydropyrido[1,2-a]indol-8-methanol

a) In einer Glove-Box (Sauerstoffgehalt <1 ppm) werden in einem 50 ml-Glaskolben 9,2 mg (0,0226 mMol) Bis-(1,5-cyclooctadien)rhodium(I)-tetrafluorborat, 12,4 mg (0,0226 mMol) (R)-BIPHEMP und 14,6 mg (0,045 mMol) Tetrabutylammoniumbromid in 20 ml Toluol suspendiert. Die Suspension wird anschliessend 60 Minuten gerührt, wobei sich eine klare, orange Lösung (die Katalysatorlösung) bildet.
b) In einer Glove-Box (Sauerstoffgehalt <1 ppm) wird ein 400 ml-Autoklav (Hastelloy C4) mit 22,5 g (0,113 Mol) 6,7-Dihydropyrido[1,2-a]indol-8-methanol, der oben erwähnten Katalysatorlösung und 130 ml Toluol beladen. Die Hydrierung wird bei 80°C, einem konstanten Druck von 60 bar H₂ und unter intensivem Rühren durchgeführt. Nach 16 Stunden Hydrierzeit beträgt der Umsatz gemäss GC 100%. Zum Reaktionsgemisch (brauner Kristallbrei) werden 250 ml Diäthyläther gegeben, und die Suspension wird bei 50°C gerührt, bis alles gelöst ist. Zur Kristallisation des Produktes wird im Teilvakuum auf ein Gewicht von 95 g eingedampft und die entstandene Suspension 3 Stunden bei Raumtemperatur gerührt. Die beigen Kristalle werden abfiltriert, mit kaltem Toluol gewaschen und 18 Stunden bei 40°C/20 mbar getrocknet.
   Ausbeute: 19,8 g (87,1%) (S)-6,7,8,9-Tetrahydropyrido[1,2-a]indol-8-methanol; Smp 107,5-109,0°C; DC 1 Fleck; GC 100 Flächen-%; 98,9% e.e.; [α]_{D}²⁰ - 47,2° (c = 1, CHCl₃).
   Die Mutterlauge wird durch eine Schicht von 5 g Kieselgel filtriert. Nachwaschen mit 100 ml Diäthyläther und Eindampfen des vereinigten Filtrates liefern weitere 2,44 g (10,7%) Produkt; GC 100 Flächen-%; 97,7% e.e.

### Beispiel 3

### Herstellung von (S)-6,7,8,9-Tetrahydropyrido[1,2-a]indol-8-methanol

a) In einer Glove-Box (Sauerstoffgehalt <1 ppm) werden in einem 100 ml-Messkolben 12,3 mg (0,025 mMol) Di-µ-chlor-bis-(1,5-cyclooctadien)dirhodium(I) und 27,5 mg (0,050 mMol) (R)-BIPHEMP in 100 ml Tetrahydrofuran suspendiert. Die Suspension wird anschliessend 10 Minuten gerührt, wobei sich eine klare, gelbe Lösung (die Katalysatorlösung) bildet.
b) In einer Glove-Box (Sauerstoffgehalt <1 ppm) wird der Glaseinsatz eines 30 ml-Autoklaven mit 0,20 g (1,0 mMol) 6,7-Dihydropyrido[1,2-a]indol-8-methanol, 9 ml Tetrahydrofuran und 1 ml der oben erwähnten Katalysatorlösung beladen. Die Hydrierung wird bei 80°C, einem Anfangsdruck von 60 bar H₂ und unter intensivem Rühren durchgeführt. Nach 18 Stunden Hydrierzeit berägt der Umsatz gemäss GC 99,9%. Die hellgelbe Hydrierlösung wird eingedampft, der Rückstand in Diäthyläther gelöst, und die Lösung zur Abtrennung des Katalysators durch eine Säule mit 5 g Kieselgel fliessen gelassen. Nachwaschen der Säule mit Diäthyläther, Eindampfen des Eluates und Trocknen des hellbraunen, kristallinen Rückstandes liefern 0,20 g (100%) (S)-6,7,8,9-Tetrahydropyrido[1,2-a]indol-8-methanol; GC 100 Flächen-%; 91,0% e.e.

### Beispiele 4-10

### Herstellung von (R)- oder (S)-6,7,8,9-Tetrahydro[1,2-a]indol-8-methanol

Analog dem in Beispiel 1, 2 oder 3 beschriebenen Verfahren werden Hydrierungen jeweils unter Verwendung eines Rhodium-Katalysators der Formel III' ([Rh(X¹)(Y¹)(L¹)ₘ]ₙ) durchgeführt, und zwar zwecks Umwandlung des 6,7-Dihydropyrido[1,2-a]indol-8-methanols in das (R)- und (S)-6,7,8,9-Tetrahydro[1,2-a]indol-8-methanol. Wie in den Beispielen 1-3 erfolgt die Hydrierung jeweils in Toluol bei 80°C und einem konstanten Druck von 60 bar H₂. Das Molverhältnis Substrat:Katalysator beträgt jeweils 2000. Der jeweilige Katalysator und Umsatz nach 18 Stunden gemäss GC, der enantiomere Ueberschuss (e.e.) und die Konfiguration [(R)/(S)] des Liganden Y¹ und des Produktes sind in der nachfolgenden Tabelle angegeben.

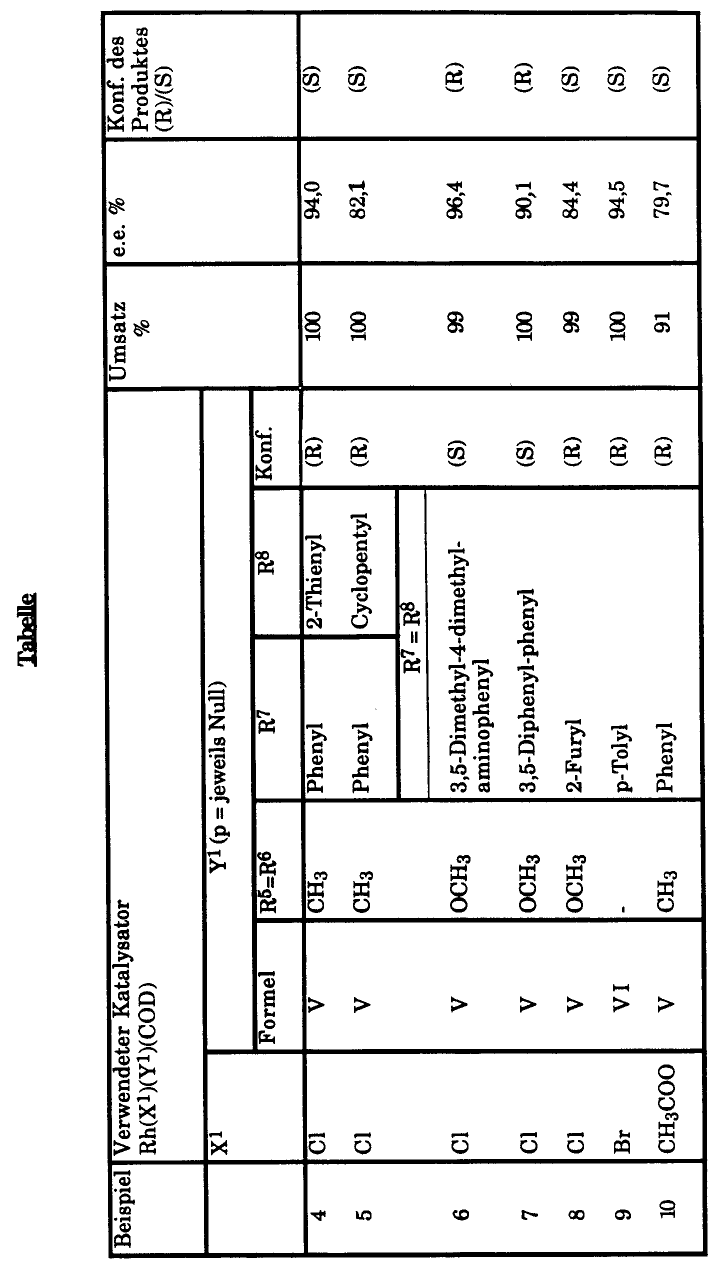

### Beispiel 11

### Herstellung von 6,7-Dihydropyrido[1,2-a]indol-8-methanol

Ein Gemisch von 18 g (0,07 Mol) 6,7-Dihydro-9-hydroxypyrido[1,2-a]indol-8-carbonsäure-äthylester und 13,3 g (0,21 Mol) Ammoniumformiat in 300 ml Aethanol wird unter einer Stickstoffatmosphäre mit 2,2 g 10%-igem Palladium/Kohle versetzt und vier Stunden auf Rückflusstemperatur erhitzt. Man kühlt die resultierende Suspension auf Raumtemperatur ab, filtriert sie und wäscht den Feststoff mit 50 ml Aethanol und zweimal mit je 50 ml Methylenchlorid. Dann dampft man das Filtrat und die Spülungen ein und nimmt den Rückstand in ein Gemisch von Aethylacetat und Wasser (5:4, insgesamt 450 ml) auf. Die organische Phase wird mit 100 ml Natriumchloridlösung gewaschen, über wasserfreiem Natriumsulfat getrocknet und zur Trockene eingedampft. Auf diese Weise erhält man 17,5 g (96%) 6,7,8,9-Tetrahydro-9-hydroxypyrido[1,2-a]indol-8-carbonsäure-äthylester als beigen Festkörper, Smp. 116-120°C.

Eine Lösung von 75 g (0,29 Mol) 6,7,8,9-Teträhydro-9-hydroxypyrido[1,2-a]indol-8-carbonsäure-äthylester in 1000 ml Pyridin wird innert 10 Minuten tropfenweise mit 59,2 g (0,58 Mol) Essigsäureanhydrid versetzt. Man erhitzt das Reaktionsgemisch 24 Stunden bei 50°C und dampft anschliessend das Lösungsmittel ab. Der Rückstand wird in ein Gemisch von Aethylacetat und Wasser (2:1, insgesamt 750 ml) aufgenommen und die organische Phase mit 250 ml Wasser und zweimal mit je 100 ml 1N Salzsäure gewaschen, über wasserfreiem Natriumsulfat getrocknet und zur Trockene eingedampft. Auf diese Weise erhält man 87 g (100%) 9-Acetoxy-6,7,8,9-tetrahydropyrido[1,2a]indol-8-carbonsäure-äthylester als hellgelben Festkörper, Smp. 98-100°C.

Man versetzt eine Lösung von 9,93 g (0,033 Mol) 9-Acetoxy-6,7,8,9-tetrahydropyrido[1,2-a]indol-8-carbonsäure-äthylester in 150 ml Toluol mit 8,3 ml (0,066 Mol) 1,1,3,3-Tetramethylguanidin und erhitzt das Reaktionsgemisch 17 Stunden bei 80°C. Dann wird die resultierende Lösung auf Raumtemperatur abgekühlt, der Reihe nach dreimal mit je 50 ml 1N Salzsäure, 50 ml wässriger Natriumbicarbonatlösung und 50 ml Natriumchloridlösung gewaschen, über wasserfreiem Natriumsulfat getrocknet und zur Trockene eingedampft. Nach Triturieren des Rückstandes mit n-Hexan erhält man 6,64 g (83%) 6,7-Dihydropyrido[1,2-a]indol-8-carbonsäure-äthylester als hellgelben Festkörper, Smp. 77-79°C.

Eine Lösung von 26 g (0,108 Mol) 6,7-dihydropyrido[1,2-a]indol-8-carbonsäure-äthylester in 850 ml trockenem Tetrahydrofuran wird innert 1 Stunde unter einer Stickstoffatmosphäre tropfenweise mit einer 1 M Lösung von Diisobutylaluminiumhydrid in Tetrahydrofuran (220 ml, 0,22 Mol) versetzt. Man rührt das Reaktionsgemisch 17 Stunden und tropft anschliessend 50 ml Methanol ein. Nach Zugabe von 1000 ml Wasser und 500 ml Toluol wird das Gemisch filtriert und der wässrige Teil des Filtrats abgetrennt und mit 250 ml Toluol extrahiert. Die vereinigten organischen Phasen werden dann über wasserfreiem Natriumsulfat getrocknet und zur Trockene eingedampft. Man erhält 19,8 g (92%) 6,7-Dihydropyrido[1,2-a]indol-8-methanol als beigen Festkörper, Smp. 139-142°C.

## Patentansprüche

1. Verfahren zur Herstellung von Verbindungen der allgemeinen Formel 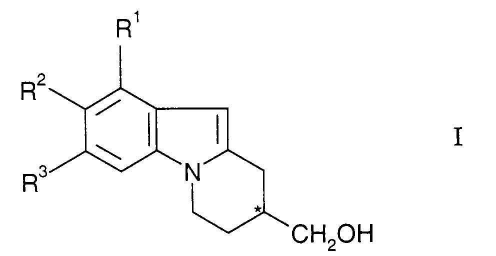
worin R¹, R² und R³ unabhängig voneinander Wasserstoff, Halogen, C₁-C₇ Alkyl, C₁-C₇ Halogenalkyl, Hydroxy, C₁-C₇ Alkoxy, C₁-C₇ Alkylthio, C₁-C₇ Alkylsulfinyl, C₁-C₇ Alkylsulfonyl, Nitro, Amino oder Acylamino bedeuten und * das chirale Zentrum bezeichnet,
in der (S)- oder (R)-Form, dadurch gekennzeichnet, dass man das entsprechende 6,7-Dihydropyrido[1,2-a]indol-8-methanol der allgemeinen Formel 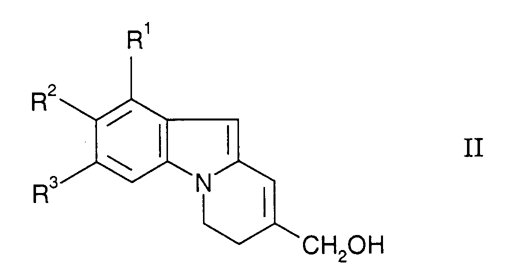
worin R¹, R² und R³ die oben angegebenen Bedeutungen besitzen,
in Gegenwart eines optisch aktiven Rhodium-Diphosphin-Komplexes asymmetrisch hydriert, wobei man als optisch aktiven Rhodium-Diphosphin-Komplex einen Komplex der allgemeinen Formel
[Rh(X¹)(Y¹)(L¹)m]n III'
verwendet, worin
X¹ Halogenid, ein Anion R⁴-COO-, ein Anion R⁴-SO₃-, 1,3-Diketonat oder gegebenenfalls
mit C₁-C₄ Alkyl und/oder Halogen einfach oder mehrfach substituiertes Phenolat,
Y¹ einen optisch aktiven atropisomeren Diphosphinliganden der allgemeinen Formel 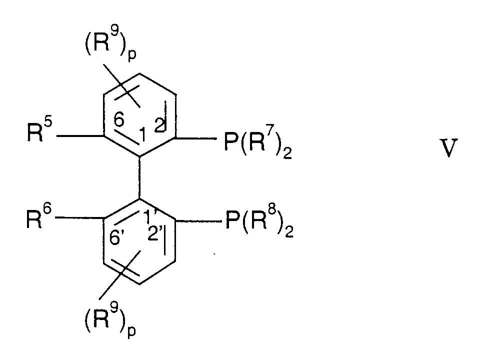 oder der allgemeinen Formel 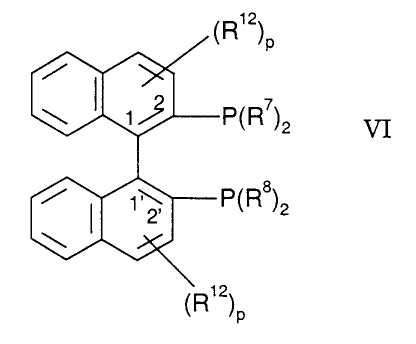
L¹, oder jedes L¹ unabhängig, einen olefinischen Liganden, ein Nitril oder ein Lösungsmittelmolekül,
m 0,1 oder 2,
n 1 oder 2,
R⁴ C₁-C₄ Alkyl, halogeniertes C₁-C₄ Alkyl oder Aryl,
R⁵ und R⁶ unabhängig voneinander C₁-C₄ Alkyl, C₁-C₄ Alkoxy, Di(C₁-C₄ Alkyl)amino, Hydroxy, geschütztes Hydroxy, Hydroxymethyl oder geschütztes Hydroxymethyl,
oder
R⁵ und R⁶ zusammen eine zweiwertige Gruppe 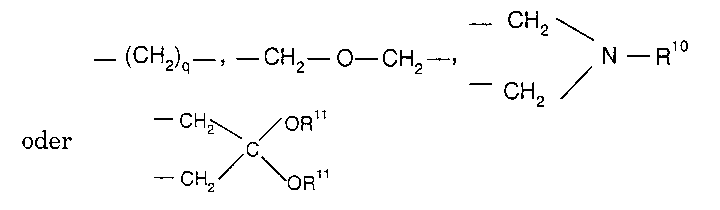
R⁷ und R⁸ unabhängig voneinander C₁-C₄ Alkyl, C₃-C₇-Cycloalkyl, gegebenenfalls substituiertes Phenyl, einen fünfgliedrigen Heteroaromaten oder eine Gruppe der Formel 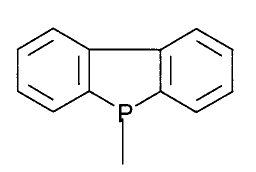
R⁹ C₁-C₄ Alkyl oder C₁-C₄ Alkoxy,
R¹⁰ C₁-C₄ Alkyl, gegebenenfalls substituiertes Phenyl oder gegebenenfalls substituiertes Benzyl,
R¹¹ C₁-C₄ Alkyl oder beide R¹¹ zusammen Di- oder Trimethylen,
R¹² Halogen, Hydroxy, Methyl, Aethyl, Amino, Acetamido, Nitro oder Sulfo,
p Null oder die Zahl 1, 2 oder 3
und q die Zahl 3, 4 oder 5 bedeuten,
wobei der Begriff 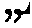 Acyl" in 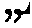 Acylamino" eine Gruppe bezeichnet, die von einer Alkankarbonsäure mit bis zu 7 Kohlenstoffatomen abgeleitet ist,
der Begriff 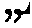 1,3-Diketonat" eine Verbindung der Formel R¹⁵-CO-CH₂-CO-R¹⁵ bezeichnet, in denen R¹⁵ C₁-C₄ Alkyl oder Aryl gemäss nachstehender Definition ist,
der Begriff 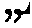 Aryl" Phenyl, Biphenyl oder Napthyl bedeutet, das unsubstituiert oder einfach oder mehrfach mit gleichen oder verschiedenen C₁-C₄ Alkylgruppen und/oder Halogenatomen substituiert ist,
der Begriff 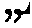 geschütztes Hydroxy" oder 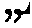 geschütztes Hydroxymethyl" mit ätherbildenden Gruppen geschützte Hydroxygruppen bedeutet,
der Begriff 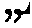 ggf. Substituiertes Phenyl" bzw. 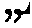 ggf. Substituiertes Benzyl" bezüglich des Substituenten des Phenylteils Fluor, C₁-C₄ Alkyl, C₁-C₄ Alkoxy, Di(C₁-C₄ Alkyl)amino, Di(C₁-C₄ Alkyl)silyl und Phenyl bedeutet,
der Begriff 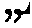 fünfgliedriger Heteroaromat" für einen Substituenten der Formel 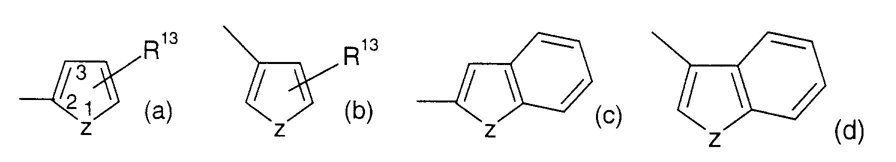
steht,worin Z Sauerstoff, Schwefel oder NR¹⁴; R¹³ C₁-C₄ Alkyl oder C₁-C₄ Alkoxy und R¹⁴ C₁-C₄ Alkyl bedeuten.

2. Verfahren nach Anspruch 1 dadurch gekennzeichnet, dass man ausgehend von 6,7-Dihydropyrido[1,2-a]indol-8-methanol das (S)-6,7,8,9-Tetrahydropyrido[1,2-a]indol-8-methanol herstellt.

3. Verbindungen der allgemeinen Formel 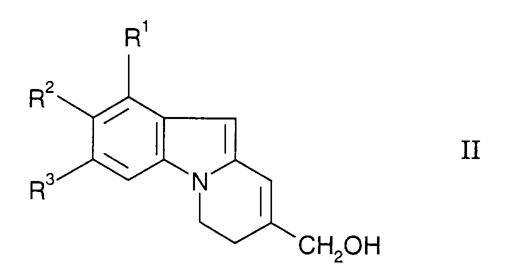
worin R¹, R² und R³ unabhängig voneinander die Bedeutung gemäss Anspruch 1 haben.

## Claims

1. A process for the manufacture of compounds of the general formula 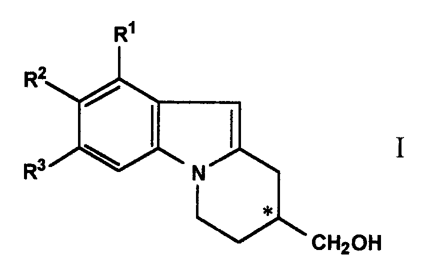
wherein R¹, R² and R³ each independently signify hydrogen, halogen, C₁-C₇ alkyl, C₁-C₇ haloalkyl, hydroxy, C₁-C₇ alkoxy, C₁-C₇ alkylthio, C₁-C₇ alkylsulphinyl, C₁-C₇ alkylsulphonyl, nitro, amino or acylamino and * denotes the chiral centre,
in the (S)- or (R)-form, characterized by asymmetrically hydrogenating the corresponding 6,7-dihydropyrido [1,2-a]indole-8-methanol of the general formula 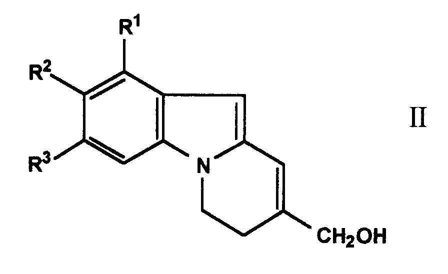
wherein R¹, R² and R³ have the significances given above,
in the presence of an optically active rhodium-diphosphine complex, with the rhodium-diphosphine complex used being a complex of the general formula
[Rh(X¹)(Y¹)(L¹)m]n III'
wherein
X¹ signifies halide, an anion R⁴-COO⁻, an anion R⁴-SO₃⁻, 1,3-diketonate or phenolate optionally mono- or multiply-substituted with C₁-C₄ alkyl and/or halogen,
Y¹ signifies an optically active atropisomeric diphosphine ligand of the general formula 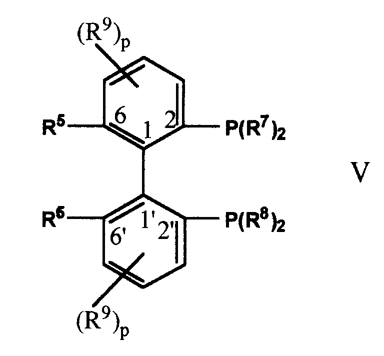 or of the general formula 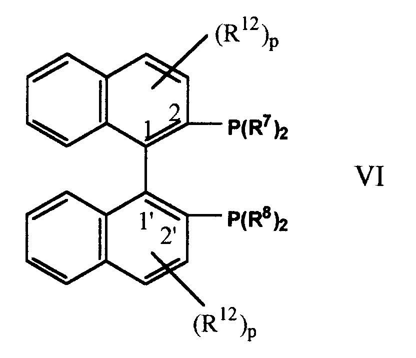
L¹, or each L¹ independently, signifies an olefinic ligand, a nitrile or a solvent molecule,
m signifies 0, 1 or 2,
n signifies 1 or 2,
R⁴ signifies C₁-C₄ alkyl, halogenated C₁-C₄ alkyl or aryl,
R⁵ and R⁶ each independently signify C₁-C₄ alkyl, C₁-C₄ alkoxy, di(C₁-C₄ alkyl)amino, hydroxy, protected hydroxy, hydroxymethyl or protected hydroxymethyl
or
R⁵ and R⁶ together signify a divalent group 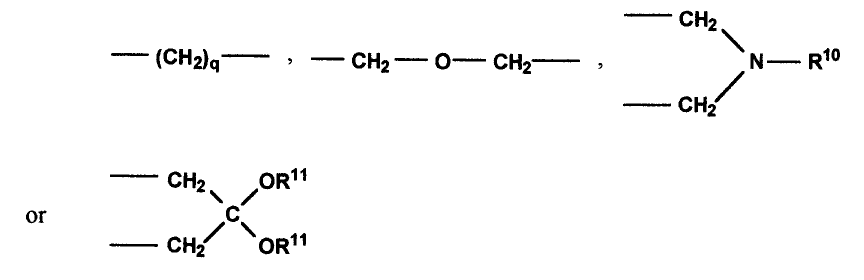
R⁷ and R⁸ each independently signify C₁-C₄ alkyl, C₃₋₇-cycloalkyl, optionally substituted phenyl, a five-membered heteroaromatic or a group of the formula 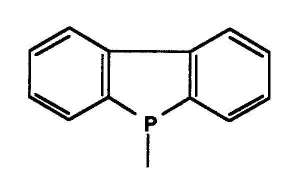
R⁹ signifies C₁-C₄ alkyl or C₁-C₄ alkoxy,
R¹⁰ signifies C₁-C₄ alkyl, optionally substituted phenyl or optionally substituted benzyl,
R¹¹ signifies C₁-C₄ alkyl or both R¹¹'s together signify di- or trimethylene,
R¹² signifies halogen, hydroxy, methyl, ethyl, amino, acetamido, nitro or sulpho,
p signifies zero or the number 1, 2 or 3
and q signifies the number 3, 4 or 5,
whereby the term "acyl" in "acylamino" denotes a group which is derived from an alkanecarboxylic acid with up to 7 carbon atoms,
the term ''1,3-diketonate" denotes a compound of the formula R¹⁵-CO-CH₂-CO-R¹⁵ in which R¹⁵ is C₁-C₄ alkyl or aryl in accordance with the following definition,
the term "aryl" signifies phenyl, biphenyl or naphthyl which is unsubstituted or mono- or multiply-substituted with the same or different C₁-C₄ alkyl groups and/or halogen atoms,
the term "protected hydroxy" or "protected hydroxymethyl" signifies hydroxy groups protected with ether-forming groups,
the term "optionally substituted phenyl" or "optionally substituted benzyl" signifies with respect to the substituents on the phenyl moiety fluorine, C₁-C₄ alkyl, C₁-C₄ alkoxy, di(C₁-C₄ alkyl)amino, di(C₁-C₄ alkyl)silyl and phenyl,
the term "five-membered heteroaromatic" stands for a substituent of the formula 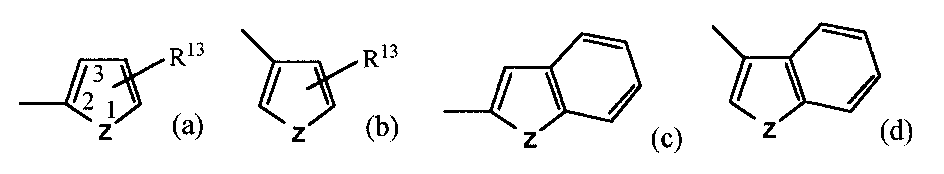
wherein Z signifies oxygen, sulphur or NR¹⁴; R¹³ signifies C₁-C₄ alkyl or C₁-C₄ alkoxy and R¹⁴ signifies C₁-C₄ alkyl.

2. A process according to claim 1, characterized in that (S)-6,7,8,9-tetrahydropyrido [1,2-a]indole-8-methanol is manufactured starting from 6,7-dihydropyrido-[1,2-a]indole-8-methanol.

3. Compounds of the general formula 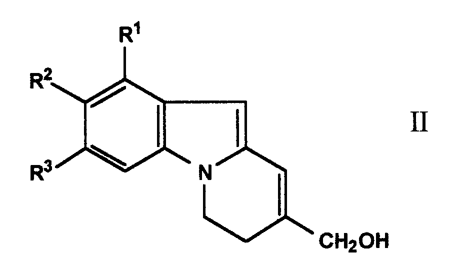
wherein R¹, R² and R³ each independently have the significance in accordance with claim 1.

## Revendications

1. Procédé de préparation de composés de formule générale 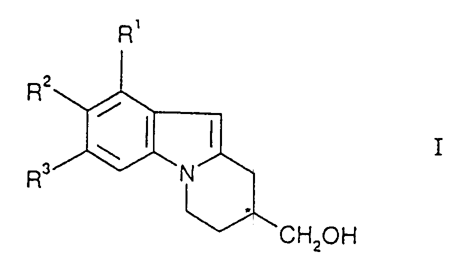
dans laquelle R¹, R² et R³ représentent chacun indépendamment des autres un atome d'hydrogène, un groupe halogéno, alkyle en C₁-C₇, halogènalkyle en C₁-C₇, hydroxy, alcoxy en C₁-C₇, alkylthio en C₁-C₇, alkylsulfinyle en C₁-C₇, alkylsulfonyle en C₁-C₇, nitro, amino ou acylamino, et * désigne le centre chiral,
sous forme (S) ou (R), caractérisé en ce qu'on soumet à une hydrogénation asymétrique le 6,7-dihydropyrido-[1,2-a]indole-8-méthanol correspondant de formule générale 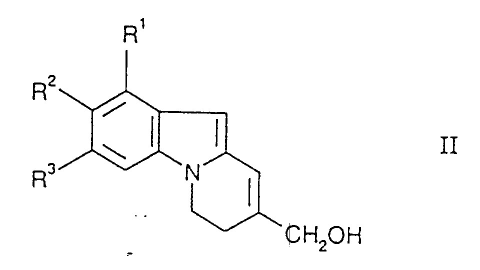 dans laquelle R¹, R² et R³ ont les significations données ci-dessus, en présence d'un complexe optiquement actif de rhodium-diphosphine, où on utilise, en tant que complexe optiquement actif de rhodium-diphosphine, un complexe de formule générale
[Rh(X¹)(Y¹)(L¹)m]n III
dans laquelle
X¹ est un halogénure, un anion R⁴-COO-, un anion R⁴-SO₃⁻, un 1,3-dicétonate ou un phénolate éventuellement substitué une ou plusieurs fois par des substituants alkyle en C₁-C₄ et/ou halogéno,
Y¹ est un ligand diphosphine atropisomère optiquement actif de formule générale 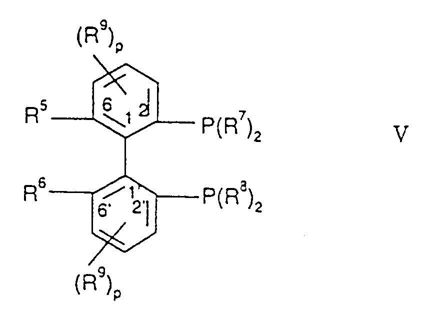 ou de formule générale 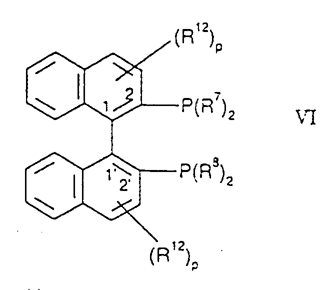
L¹, ou chaque L¹ indépendamment des autres, représente un ligand oléfinique, un nitrile ou une molécule de solvant,
m vaut 0, 1 ou 2,
n vaut 1 ou 2,
R⁴ est un groupe alkyle en C₁-C₄, alkyle en C₁-C₄ halogéné ou aryle,
R⁵ et R⁶ représentent chacun indépendamment de l'autre un groupe alkyle en C₁-C₄, alcoxy en C₁-C₄, di(alkyle en C₁-C₄)amino, hydroxy, hydroxy protégé, hydroxyméthyle ou hydroxyméthyle protégé,
ou bien
R⁵ et R⁶ forment ensemble un groupe divalent 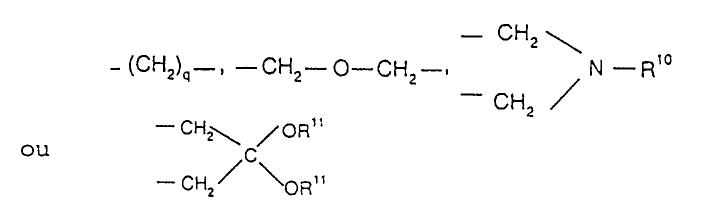
R⁷ et R⁸, indépendamment l'un de l'autre, représentent chacun un groupe alkyle en C₁-C₄, cycloalkyle en C₃-C₇, phényle éventuellement substitué, un composé hétéroaromatique à cinq chaînons, ou un groupe de formule 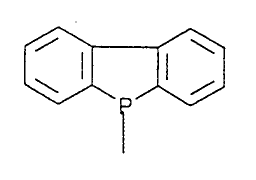
R⁹ est un groupe alkyle en C₁-C₄ ou alcoxy en C₁-C₄,
R¹⁰ est un groupe alkyle en C₁-C₄, phényle éventuellement substitué ou benzyle éventuellement substitué,
R¹¹ est un groupe alkyle en C₁-C₄, ou les deux radicaux R¹¹ forment ensemble un radical di- ou triméthylène,
R¹² est un groupe halogéno, hydroxy, méthyle, éthyle, amino, acétamido, nitro ou sulfo,
p vaut zéro ou est le nombre 1, 2 ou 3,
et q représente le nombre 3, 4 ou 5,
où le terme "acyle", dans "acylamino", désigne un groupe qui dérive d'un acide alcanecarboxylique ayant jusqu'à 7 atomes de carbone,
le terme "1,3-dicétonate" désigne un composé de formule R¹⁵-CO-CH₂-CO-R¹⁵, dans laquelle R¹⁵ est un groupe alkyle en C₁-C₄ ou aryle selon la définition ci-après,
le terme "aryle" désigne le groupe phényle, biphényle ou naphtyle, qui est non-substitué, ou est une ou plusieurs fois substitué par des groupes alkyle en C₁-C₄ et/ou des atomes d'halogène identiques ou différents,
l'expression "hydroxy protégé" ou "hydroxyméthyle protégé" désigne des groupes hydroxy protégés par des groupes éthérifiables,
dans les expressions "phényle éventuellement substitué" et "benzyle éventuellement substitué", le substituant du fragment phényle représente un groupe fluoro, alkyle en C₁-C₄, alcoxy en C₁-C₄, di(alkyle en C₁-C₄)amino, di(alkyle en C₁-C₄) silyle, et phényle,
l'expression "composé hétéroaromatique à cinq chaînons" désigne un substituant de formule 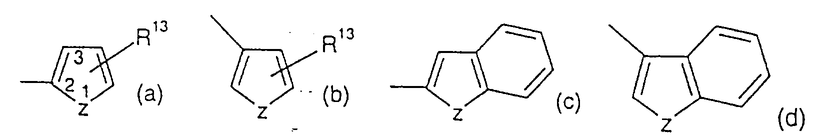
dans laquelle Z est un atome d'oxygène ou de soufre, ou NR¹⁴ ; R¹³ est un groupe alkyle en C₁-C₄ ou alcoxy en C₁-C₄, et R¹⁴ est un groupe alkyle en C₁-C₄.

2. Procédé selon la revendication 1, caractérisé en ce que, en partant de 6,7-dihydropyrido[1,2-a]indole-8-méthanol, on prépare le (S)-6,7,8,9-tétrahydropyrido[1,2-a] indole-8-méthanol.

3. Composés de formule générale 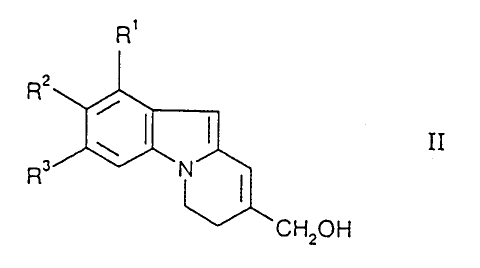
dans laquelle R¹, R² et R³ ont chacun indépendamment des autres les significations selon la revendication 1.
